# EUROPEAN PATENT SPECIFICATION

(11) **EP 3 694 444 B1**
(45) Date of publication and mention of the grant of the patent: **25.11.2026**
(21) Application number: 18800786.8
(22) Date of filing: 09.10.2018
(51) Int. Cl.: A61F 2/07

(54) **MATCHED STENT COVER**
ANGEPASSTE STENTABDECKUNG
REVÊTEMENT D'ENDOPROTHÈSE ADAPTÉ

(30) Priority: 09.10.2017 US 201762569805 P
(43) Date of publication of application: 19.08.2020
(60) Divisional application: 26210371.6
(73) Proprietor: W. L. Gore & Associates, Inc., Newark, DE 19711 (US)
(72) Inventor: SILVERMAN, James D., Newark 19711 (US); IRWIN, Crag W., Newark Delaware 19711 (US); SKELTON, Tyson J., Newark Delaware 19711 (US)
(74) Representative: HGF
(86) International application number: PCT/US2018/054915
(87) International publication number: WO 2019/074869

(56) References cited:
- WO-A1-2013/074990
- WO-A1-2017/038145
- WO-A1-95/09586
- US-A- 5 064 435
- US-A- 6 110 198
- US-A- 6 161 399
- US-A1- 2003 055 494
- US-A1- 2017 105 854

## Description

### CROSS-REFERENCE TO RELATED APPLICATIONS

This application claims the benefit of U.S. Provisional Application No. 62/569,805, filed October 9, 2017.

### BACKGROUND

Medical stents are generally known. Stents, in combination with coverings, also can be used for the endovascular repair of aneurysms, an abnormal widening or ballooning of a portion of a body lumen which can be related to weakness in the wall of the body lumen. Various stent designs are known in the art. Stents typically are tubular, and are expandable or self-expand from a relatively small diameter to a larger diameter.

Braided stents are popular for bare metal constructs. Covering a braided stent has challenges in that the covering will wrinkle, stretch, or tear if it does not move in tandem with the wires.

WO 95/09586 A1 describes a porous, self-expanding intraluminal composite prosthesis comprised of a rigid reinforcing component and sealing component.

US 6161399 A describes a wire reinforced fabric stent having improved elasticity and axial elongation together with a method of weaving.

### SUMMARY

The invention is defined in appended claim 1. Preferred embodiments of the invention are defined in the depended claims.

### BRIEF DESCRIPTION OF THE DRAWINGS

FIG. 1 shows an example stent, consistent with various aspects of the present disclosure.
FIG. 2 shows an example stent and tubular member in a non-deformed configuration, consistent with various aspects of the present disclosure.
FIG. 3 shows another example stent and tubular member in a deformed configuration, consistent with various aspects of the present disclosure.
FIG. 4A shows another example tubular member in an undeformed state, consistent with various aspects of the present disclosure.
FIG. 4B shows another example tubular member in a deformed state, consistent with various aspects of the present disclosure.
FIG. 5 shows another example stent and tubular member, consistent with various aspects of the present disclosure.
FIG. 6 shows an example laser-cut stent, consistent with various aspects of the present disclosure.
FIG. 7A shows an image of an example tubular member having a strength aligned in a first direction, consistent with various aspects of the present disclosure.
FIG. 7B shows a close-up view of the image of an example tubular member, in FIG. 7B, consistent with various aspects of the present disclosure.

### DETAILED DESCRIPTION

Persons skilled in the art will readily appreciate that various aspects of the present disclosure can be realized by any number of methods and apparatuses configured to perform the intended functions. It should also be noted that the accompanying figures referred to herein are not necessarily drawn to scale, but may be exaggerated to illustrate various aspects of the present disclosure, and in that regard, the figures should not be construed as limiting.

A medical device, consistent with various aspects of the present disclosure, is a device adapted to be inserted into a body and then deployed within the body. Such medical devices may be deployed within an artery or other vessel. Most generally, medical devices according to various examples assist in structurally supporting the host vessel lumen, maintaining patency through the vessel, passageway or opening, repairing vessels having an intimal flap or dissection, or isolating sections of a host vessel lumen, such as aneurysms. The medical devices may be shaped and sized and otherwise customized to fit a particular anatomy, including adjusting its length and inside diameters. The medical devices may include a stent with a framework of struts (or relatively rigid sections) and also may include a graft coupled or attached to the framework of struts.

Grafts or coverings in combination with the stent may help minimize or at least reduce the risk of introduction of emboli into a bloodstream, resist tissue encroachment into the lumen defined by the stent, reduce pressure on a weakened part of a blood vessel to reduce the risk of vessel rupture, and/or to create a conduit for attaching at least two vessels. The grafts or coverings may be made from continuous materials with no holes visible without magnification. Various grafts or coverings may be attached to the luminal (interior) or exterior surface of the stent.

In addition, the medical devices discussed herein may include braided or helical frames. The braided or helical frames may include a plurality of struts that overlap as the plurality of struts extends between ends of the medical devices. During deployment, geometry change, or other shape change of the braided frames, helical frames, or other frames, a graft or covering attached to the stent may encumber the stent's ability to expand. As discussed in further detail below, the medical devices discussed herein include tubular members (e.g., grafts or coverings) that interact and cooperate with the framework (to which the tubular members are attached) to ensure accurate deployment and functioning of the medical devices, for example with an inelastic or substantially inelastic covering or tube.

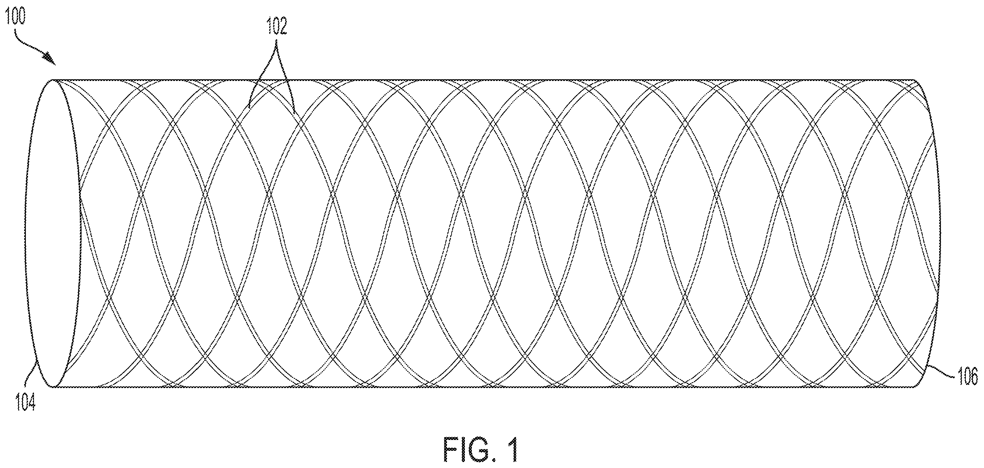

FIG. 1 shows an example stent 100, consistent with various aspects of the present disclosure. The stent 100 (or frame) includes a plurality of struts 102 that extend between a proximal end 104 and a distal end 106 of the stent 100. The stent 100 may be a support structure for an implantable medical device (e.g., an occluder, filter, or other similar device) formed by the plurality of struts 102. As shown in FIG. 1, the struts 102 form a tubular structure and it is understood that the struts 102 may form a non-cylindrical structure in certain instances. The plurality of struts 102 may overlap between the proximal end 104 and the distal end 106 of the stent 100. The plurality of struts 102 may be considered a braided stent. In addition, the plurality of struts 102 traverse a circumference of the stent 100 in lengthwise but angularly intersecting directions. The two directional sets of struts are interlaced or interwoven to form a tubular, supportive structure. In certain instances, the stent 100 may be a helical construct

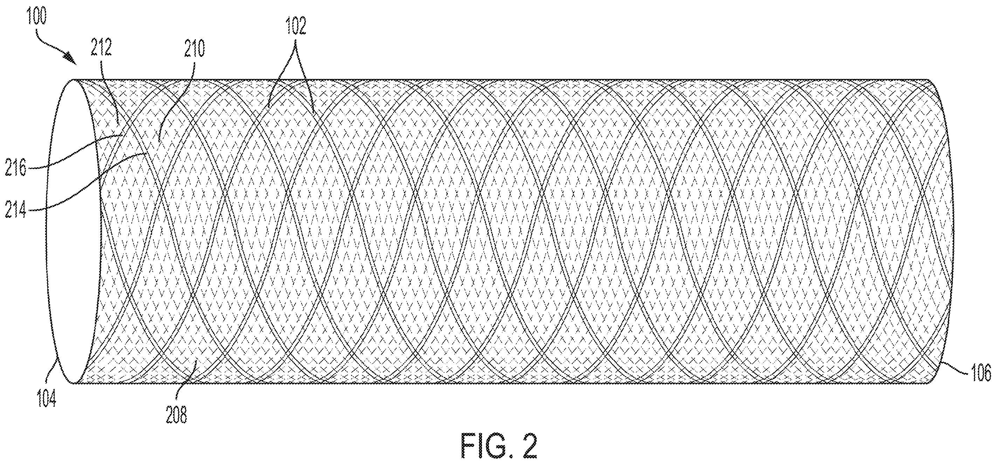

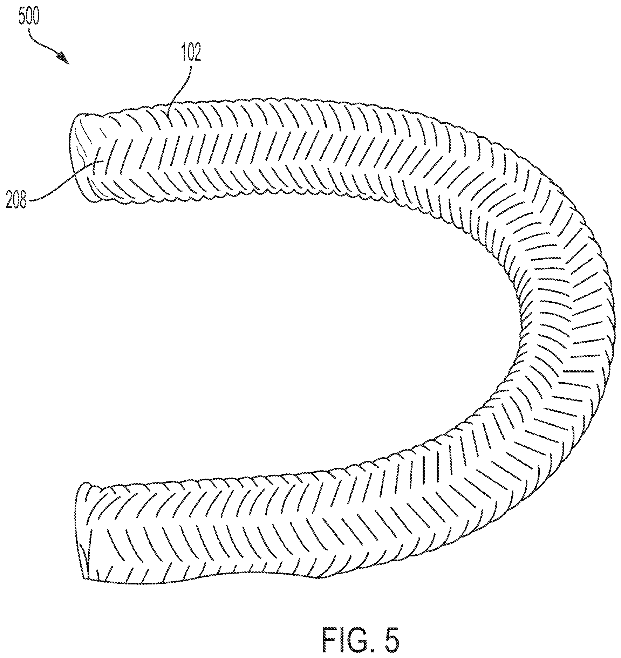

FIG. 2 shows an example stent 100 and tubular member 208 in a non-deformed configuration, consistent with various aspects of the present disclosure. The stent 100 and tubular member 208 form an implantable medical device. The tubular member 208 may be formed of an oriented polymer with a low shear strength direction (*e.g*., expanded PTFE (ePTFE)). The tubular member 208 may have a strength in one direction that is higher than a strength in another direction such that when a shear force is induced on or to the tubular member 208 (*e.g*., by way of stent 100 altering shape), the tubular member 208 is configured to reorient with the shear force. The tubular member 208 may stretch without wrinkling or tearing. The tubular member 208 is configured to move in tandem with the stent 100. In this regard, the tubular member 208 may include fibrils 210, 212, which are labeled and are represented generally in FIG. 2. It should be understood that, according to various embodiments, the fibrils 210, 212 are not readily seen by the naked eye (*e.g.*, as shown in FIG. 5). As shown, the tubular member 208 may include fibrils 210, 212 that are aligned with the plurality of struts 102. The fibrils 210, 212 optionally extend along the plurality of struts 102 and are aligned with the plurality of struts 102, according to various embodiments.

As shown in FIG. 2, the plurality of struts 102 form a frame by overlapping and helically extending between the proximal end 104 and the distal end 106 of the medical device. The plurality of struts 102 are wound such that adjacent ones of the plurality of struts 102 extend in opposite directions (*e.g.*, left handed and right handed helices). For example, a first strut 214 of the plurality of struts 102 may extend upward and toward the proximal end 104 at a location on the stent 100, whereas the second strut 216 (adjacent to the first strut 214) of the plurality of struts 102 extends upward and toward the distal end 106 of the stent 100 at that particular location. The first strut 214 represents a left handed helix, and the second strut 216 represents a right handed helix. Although the stent 100 includes a plurality of struts 102, single ones of the first strut 214 and the second strut 216 are highlighted for ease of understanding. Various other struts are shown in FIG. 2 and are arranged and extend similarly to the designated first strut 214 and designated second strut 216, and as such are similarly considered to be a first strut 214 and second strut 216.

In certain instances, the fibrils 210, 212 of the tubular member 208 are axially aligned with the plurality of struts 102. For example, tubular member may include a first set of fibrils 210 and a second set of fibrils 212 as shown in FIG. 2. The first set of fibrils 210 may be aligned with the helices of the first direction (*e.g.*, the second strut 216 of the plurality of struts 102) and the second set of fibrils 212 may be aligned with the helices of the second direction (*e.g.*, the first strut 214 of the plurality of struts 102). In certain instances, the fibrils 210, 212 extend in parallel with the plurality of struts 102. For example, the first set of fibrils 210 extend parallel to the first struts 214 and other similarly extending struts of the plurality of struts 102 shown in FIG. 2 and the second set of fibrils 212 and other similarly extending struts of the plurality of struts 102 shown in FIG. 2. Although the tubular member 208 includes multitude of fibrils 210, 212, single ones of the first set of fibrils 210 and the second set of fibrils 212 are highlighted for ease of understanding. The other fibrils shown in FIG. 2 that are arranged and extend similarly to the designated one of the first set of fibrils 210 and designated one of the second set of fibrils 212 are also considered to be, respectively, part of the first set of fibrils 210 and the second set of fibrils 212.

In certain instances, the plurality of struts 102 are braided and extend helically between the proximal end 104 and the distal end 106. The fibrils 210, 212 are configured to align with a geometry of the plurality of struts 102. For example, the fibrils 210, 212 extend at the same pitch angle at which the plurality of struts 102 extend. In addition and as noted above, the plurality of struts 102 are angled such that adjacent ones of the plurality of struts 102 extend in intersecting directions to form the braided stent 100. The first strut 214 and the first set of fibrils 212 may extend upward and toward the proximal end 104 at a location on the stent 100, whereas the second strut 216 (adjacent to the first strut 214) and the second set of fibrils 212 extends upward and toward the distal end 106 of the stent 100 at that particular location.

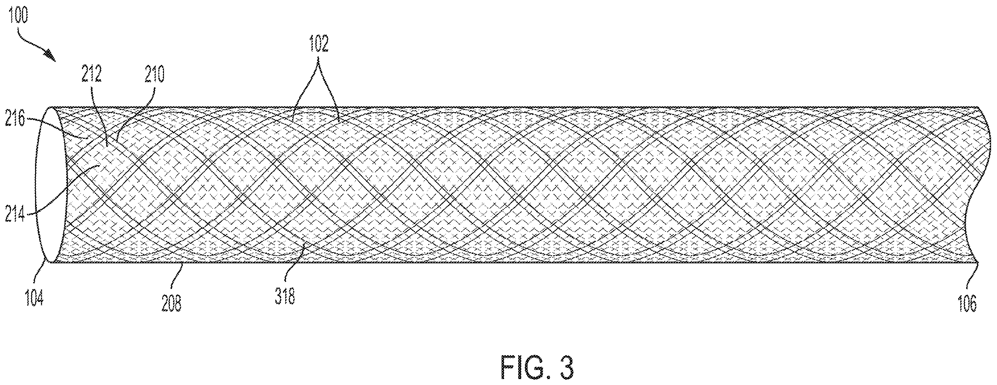

In addition to being aligned with the plurality of struts 102, the fibrils 210, 212 are configured to maintain alignment with the plurality of struts 102. The fibrils, for example, maintain alignment with the plurality of struts 102 when the stent 100 changes configuration, geometry, or shape as is described in further detail with reference to FIG. 3.

FIG. 3 shows another example stent 100 and tubular member 208 in a deformed configuration, consistent with various aspects of the present disclosure. As compared to the stent 100 and tubular member 208 shown in FIG. 2, the stent 100 and tubular member 208 have been reduced in circumference (*e.g.*, compressed) and elongated. The deformed configuration shown in FIG. 3, may be a delivery configuration for the stent 100 and tubular member 208, or a configuration of the stent 100 and tubular member 208 as the result of forces acting on the stent 100 and/or the tubular member 208 (*e.g.*, after implantation in a patient).

As noted above with reference to FIG. 2, for example, fibrils 210, 212 are configured to maintain alignment with the plurality of struts 102 in addition to being aligned with the plurality of struts 102. The fibrils, for example, maintain alignment with the plurality of struts 102 when the stent 100 changes configuration, geometry, or shape. As shown in FIG. 3, the plurality of struts 102 have reoriented as a result of being reduced in diameter. Due to the helical/braided configuration of the plurality of struts 102, the stent 100 has also elongated in comparison to the non-deformed or non-altered stent 100 shown in FIG. 2. The angle at which the plurality of struts 102 extend helically has also decreased relative to the non-deformed or non-altered stent 100 shown in FIG. 2. For example, in some examples the angle by which the plurality of struts 102 extend helically changes by greater than zero and less than 90 degrees according to various examples when the stent is transitioned from the first to the deformed state. The fibrils 210, 212 also change pitch angle in the same manner in which the plurality of struts 102 change pitch angle as shown in FIG. 2. The stent 100 and the tubular member 208 deform in conjunction with one another by way of the plurality of struts 102 and the fibrils 210, 212 re-orienting in tandem.

In certain instances, a geometry of the plurality of struts 102 changes in response to at least one of a length change of the stent 100 (*e.g.*, the frame), a circumferential change of the stent 100, or angular displacement of the struts of the stent 100. Similarly, the fibrils 210, 212 are configured to orient with the plurality of struts 102. The fibrils 210, 212 and the plurality of struts 102 orient in a direction extending toward the proximal end 104 of the stent 100 and a direction extending toward the distal end 106 of the stent 100. In certain instances, the plurality of struts 102 include a first set of struts, represented by the first strut 214, that extend at a first pitch toward the proximal end 104 and a second set of struts, represented by the second strut 216, that extend at a second pitch toward the distal end 106. Similarly, the first set of fibrils 210 extend at approximately the first pitch toward the proximal end 104 and the second set of fibrils 212 extend at approximately the second pitch toward the distal end 106. As shown in comparing FIG. 2 and FIG. 3, the first set of fibrils 210 and the first set of struts, represented by the first strut 214, alter pitch angles in coordination with one another, and the second set of fibrils 212 and the second set of struts, represented by the second strut 216, alter pitch angles in coordination with one another.

In certain instances, the first set of fibrils 210 overlap with the second set of fibrils 212 throughout the tubular member 208. In response to at least one of a length change of the stent 100, a circumferential change of the stent 100 (*e.g.*, as shown comparing FIG. 2 and FIG. 3) or angular displacement of the struts of stent 100, the fibrils 210, 212 are configured to shear (or slip) relative to one another to maintain alignment of the fibrils 210, 212 with the plurality of struts 102. The fibrils 210, 212, for example, shear within the tubular member 208 to maintain alignment with the plurality of struts 102. The tubular member 208 forms a continuous flow lumen by way of the fibrils 210, 212. The tubular member 208 may be an impermeable membrane or film.

In certain instances, the fibrils 210, 212 being configured to maintain orientation with the plurality of struts 102 allows the stent 100 to expand and contract in response to at least one of a length change of the frame, a circumferential change of the stent 100, or angular displacement of the struts of the stent 100. The fibrils 210, 212 do not otherwise encumber or restrict the ability of the stent 100 to change geometry or expand and contract. More specifically and in certain instances, the tubular member 208, by way of the fibrils 210, 212, is configured to resist residual elastic strain acting against stent 100 deformation in response to at least one of the length change of the frame, the circumferential change of the stent 100, or angular displacement of the struts of the stent. The tubular member 208 does not include residual elastic strain that acts against the stent 100 changing geometry under deformation of the stent 100.

The tubular member 208 may include non-oriented fibrils 318 in addition to the fibrils 210, 212 that are not oriented with the plurality of struts 102. The non-oriented fibrils 318 (represented by the open space between the fibrils 210, 212) may fill connect and fill space between the fibrils 210, 212. The non-oriented fibrils 318 are those fibrils of the tubular member 208 that are not oriented or aligned with primary strength of the tubular member 208.

The fibrils 210, 212 that are aligned with struts 102 of the stent 100 may have a greater strength than fibrils or nodes that connect the strength fibrils together For example, the tubular member can be made from a film that has a force to break strength direction of 1.06 kgf/cm and a force to break transverse direction strength of 0.024 kgf/cm as measured by a tensile testing machine. Other ratios of strength to transverse direction may be used dependent on application. For example a ratio of strength direction to transverse direction may be 30, 35, 40, 45, 50, 55, 60 or more. In addition, the fibrils 210, 212 that are aligned with struts 102 of the stent 100 also have a greater length greater than lengths of the fibrils that are unaligned with the struts 102 of the stent 100 and also maintain alignment with the struts 102 of the stent 100.

In certain instances, the tubular member 208 may include a single set of fibrils 210 that are aligned with struts 102 of the stent 100. The tubular member 208 may have a single strength direction oriented with the stent 100.

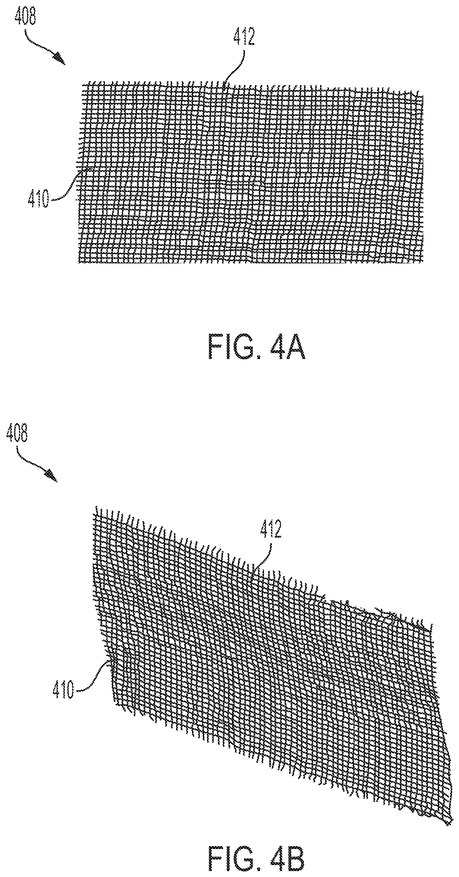

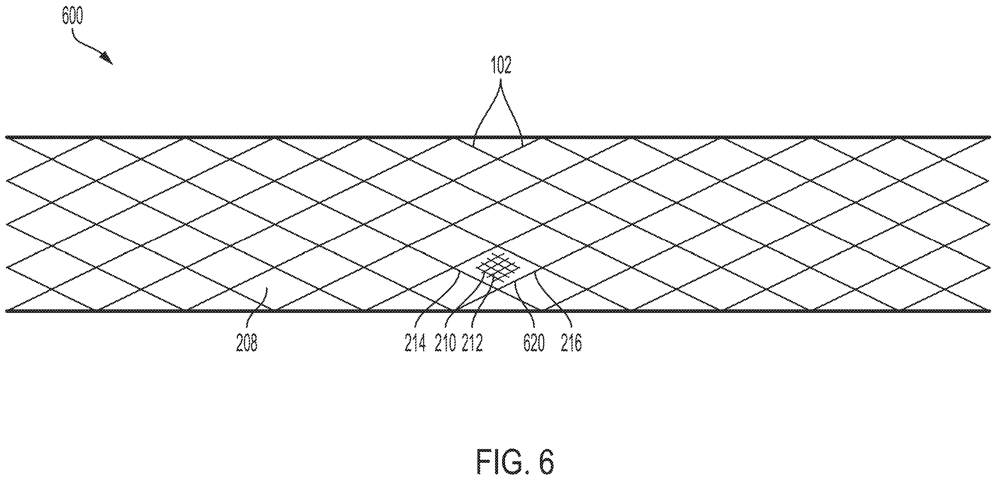

FIG. 4A shows another example tubular member 408 in an undeformed state, consistent with various aspects of the present disclosure. The tubular member 408 is a weave or knit material. The tubular member 408 may have a strength element that is aligned with one or more struts of a stent (*e.g.*, as shown in FIG. 1 or FIG. 6). The tubular member 408 may be attached to a stent that is a continuous frame, as depicted in FIG. 1 or FIG. 6 or a plurality of discrete rings to form a stent frame. In addition, the tubular member r408 may be configured to maintain orientation of the strength element with the strut on response to a force applied to the stent. The strength element of the tubular member 408 is configured to maintain orientation in response to a force acting on the tubular member 408.

As shown in FIG. 4A, the tubular member 408 member includes a series or woven or knit filaments, threads, or fibers 410, 412. The filaments, threads, or fibers 410, 412 may form the strength element. In addition, the tubular member 408 may have a strength in one direction that is higher than a strength in another direction such that when a shear force is induced on or to the tubular member 408 (*e.g.,* by way of stent 100, to which the tubular member 408 is attached, altering shape), the tubular member 408 is configured to reorient with the shear force as shown in FIG. 4B.

As shown in FIG. 4B, the tubular member 408 may stretch without wrinkling or tearing. The tubular member 408 is configured to move in tandem with the stent 100. The tubular member 408 is oriented along with a stent 100, to which the tubular member 408 is coupled to or attached to, maintain orientation of the strength element with the at least one strut in response to a force applied to the frame.

FIG. 5 shows another example stent 100 and tubular member 208, consistent with various aspects of the present disclosure. In certain instances, the tubular member 208 is coupled to the stent 100, having a plurality of struts 102, to form a medical device 500. In certain instances, the tubular member 208 may be formed of expanded PTFE (ePTFE) and attached to the stent 100 using fluorinated ethylene propylene (FEP). The tubular member 208 may be arranged on one side or both sides of the stent 100. The tubular member 208 is attached to the stent 100, in certain instances, such that fibrils contained in the tubular member 208 are aligned with struts that form the stent 100.

The illustrative medical device shown in FIG. 5 is not intended to suggest any limitation as to the scope of use or functionality of embodiments as discussed throughout this disclosure. Neither should the illustrative system be interpreted as having any dependency or requirement related to any single component or combination of components illustrated therein. For example, in various embodiments, the illustrative medical device 500 may have fibrils that are continued to maintain orientation with a plurality of struts of the stent 100 as described with reference to FIG. 1-3. In addition, the tubular member 208 may include a primary strength oriented with the plurality of struts as discussed with reference to FIG. 4.

FIG. 6 shows an example laser-cut stent 600, consistent with various aspects of the present disclosure. The stent 600 may include a plurality of diamond shaped cells 620 (one of which is highlighted in FIG. 6), although the cells 620 may have different shapes such as chevron or rectangle. The laser-cut stent could be a continuous frame as depicted or a plurality of discrete rings to form a stent frame. In addition, the laser-cut stent 600 may include a tubular member 208 attached thereto. As discussed in further detail above, the tubular member 208 may be formed of a polymer with a low shear strength direction (*e.g*., expanded PTFE (ePTFE)). The tubular member 208 may have a strength in one direction that is higher than a strength in another direction such that when a shear force is induced on or to the tubular member 208 (*e.g.*, by way of stent 600 altering shape), the tubular member 208 is configured to reorient with the shear force. The tubular member 208 may stretch without wrinkling or tearing. The tubular member 208 is configured to move in tandem with the stent 600. In this regard, the tubular member 208 may include fibrils 210, 212, which are labeled and are represented generally in FIG. 6.

As shown in FIG. 6, the stent 600 includes overlapping or crossing struts 102. Although the stent 600 includes a plurality of struts 102, single ones of a first strut 214 and a second strut 216 are highlighted for ease of understanding. Various other struts are shown in FIG. 2 and are arranged and extend similarly to the designated first strut 214 and designated second strut 216, and as such are similarly considered to be a first strut 214 and second strut 216. The fibrils 210, 212 of the tubular member 208 are axially aligned with the plurality of struts 102. For example, tubular member may include a first set of fibrils 210 and a second set of fibrils 212 as shown in FIG. 2. The first set of fibrils 210 may be aligned with the first strut 214 of the plurality of struts 102, and the second set of fibrils 212 may be aligned with the second strut 216 of the plurality of struts 102. In certain instances, the fibrils 210, 212 extend in parallel with the plurality of struts 102. Although the tubular member 208 includes multitude of fibrils 210, 212, single ones of the first set of fibrils 210 and the second set of fibrils 212 are highlighted for ease of understanding. The other fibrils of the tubular member 208 that are arranged and extend similarly to the designated one of the first set of fibrils 210 and designated one of the second set of fibrils 212 are also considered to be, respectively, part of the first set of fibrils 210 and the second set of fibrils 212.

In addition to being aligned with the plurality of struts 102, the fibrils 210, 212 are configured to maintain alignment with the plurality of struts 102. The fibrils, for example, maintain alignment with the plurality of struts 102 when the stent 100 changes configuration, geometry, or shape.

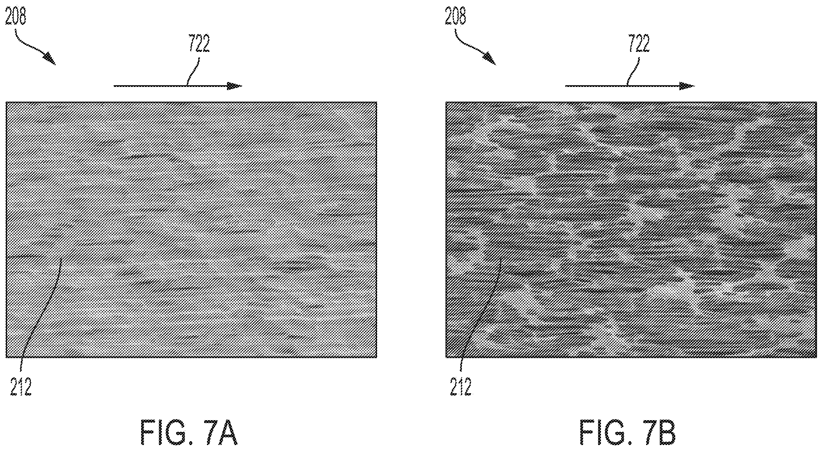

FIG. 7A shows an image of an example tubular member 208 having a strength aligned in a first direction 722, consistent with various aspects of the present disclosure. The strength may be associated with fibrils 212 within the tubular member 208. In addition, the strength of the tubular member 208 may be aligned with a strut of a stent to which the tubular member 208 is attached as discussed in detail above. The tubular member 208 being attached to a stent and aligns the strength of the tubular member 208 with a strut in the first direction 722. As a result, the tubular member 208 is configured to bias the strut in the first direction 722.

FIG. 7B shows a close-up view of the image of an example tubular member 208, in FIG. 7B, consistent with various aspects of the present disclosure.

Suitable materials for use in in the tubular member 208 may include, without limitation, fluoropolymers (especially polytetrafluoroethylene (PTFE) and fluorinated ethylene propylene (FEP)), polyethylenes, polyethylene terephthalate (PET), nylon, polyurethane, polypropylene, polyester, polyimide, etc., as well as composite materials combining these and/or other materials to achieve the desired strength and compliance characteristics. Expanded PTFE (ePTFE) is believed to be most preferred for many applications.

Depending on applications, tubular members of the present disclosure may be constructed from a continuous material, such as continuous films, tapes, or sheets of materials. Alternatively, the tubular members may include discontinuous structures, such as sheets or tapes that include holes or slits therein, or even materials formed from weaves, knits, or other open structures.

Consistent with various aspects of the present disclosure, frames discussed herein may be made from a variety of materials. These materials comprise metals, such as nitinol, stainless steel, tantalum, titanium, tungsten, gold, platinum, iridium, rhodium and alloys thereof or pyrolytic carbon. Other materials comprise polymers such as polyurethane, high density polyethylene, polypropylene, and poly(dimethyl siloxane). Further still, the frames may be formed from biocompatible polymers that are bio-resorbable (e.g., bio-erodible or biodegradable). Bio-resorbable materials are preferably selected from the group consisting of any hydrolytically degradable and/or enzymatically degradable biomaterial. Examples of suitable degradable polymers include, but are not limited to, polyhydroxybutyrate/polyhydroxyvalerate copolymers (PHV/PHB), polyesteramides, polylactic acid, hydroxy acids (i.e. lactide, glycolide, hydroxybutyrate), polyglycolic acid, lactone based polymers, polycaprolactone, poly(propylene fumarate-co-ethylene glycol) copolymer (aka fumarate anhydrides), polyamides, polyanhydride esters, polyanhydrides, polylactic acid/polyglycolic acid with a calcium phosphate glass, polyorthesters, silk-elastin polymers, polyphosphazenes, copolymers of polylactic acid and polyglycolic acid and polycaprolactone, aliphatic polyurethanes, polyhydroxy acids, polyether esters, polyesters, polydepsidpetides, polysaccharides, polyhydroxyalkanoates, and copolymers thereof. Further still, the tubes may be formed of a polycarbonate material, such as, for example, tyrosine-derived polycarbonates, tyrosine-derived polyarylates, iodinated and/or brominated tyrosine-derived polycarbonates, iodinated brominated tyrosine-derived polyarylates polyhydroxy acids, polyorthoesters, polyether esters, polyesters, polyamides, polyesteramides, polydepsidpetides, aliphatic polyurethanes, polysaccharides, polyhydroxyalkanoates, and copolymers thereof.

Additionally, the frames could be comprised of any number of other polymers. In another embodiment, metals and polymers may be used to fabricate said tube in a composite, laminate reinforced material, or one that is simply coated with the material. Depending on desired characteristics, tubes may be constructed of materials with specific attributes. For example, in applications where the tube will be expanded and must remain so with little or no creep or re-constriction (that is it must "lock in place"), plastically deformable materials may be chosen for monolithic constructs. Conversely, should a tube need to remain compliant, meaning remaining capable of some degree of radial re-contraction and re-expansion, elastic materials may be chosen. It will be recognized that combining materials with different functional or behavioral attributes may be effected in selected instances. The configuration of the tubes of the invention may be varied to produce selected benefits. In one embodiment, the components making up the frames (the struts) are asymmetrically wrapped along the entire length of the frame. However, in other embodiments, asymmetrically-wrapped frames can be interspersed and connected to torsionally-stable symmetrically-wrapped tube sections, the latter serving to transmit torque.

Persons skilled in the art will readily appreciate that various aspects of the present disclosure can be realized by any number of methods and apparatus configured to perform the intended functions. It should also be noted that the accompanying figures referred to herein are not necessarily drawn to scale, but may be exaggerated to illustrate various aspects of the present disclosure, and in that regard, the figures should not be construed as limiting.

## Claims

1. An implantable medical device comprising:
a frame (100) having a plurality of struts (102) that are braided and extend helically between a proximal end (104) and a distal end (106) of the frame; and
a continuous film formed of expanded polytetrafluoroethylene (ePTFE) defining a tubular member (208) attached to the frame (100), and including fibrils (210, 212) that are configured to coincide with a geometry of the plurality of struts, wherein the fibrils (210, 212) include strength fibrils that are aligned and extending in parallel with the plurality of struts (102) and non-oriented fibrils (318) that are not aligned with the plurality of struts (102) and connect the strength fibrils (210, 212) together, the continuous film having a force to break strength direction and a force to break transverse direction and a ratio of strength direction to transverse direction of 30 or more such that the strength fibrils (210, 212) are configured to shear within the tubular member (208) to maintain alignment with the plurality of struts (102) in response to at least one of a length change of the frame (100) and a circumferential change of the frame (100).

2. The implantable medical device of claim 1, wherein the strength fibrils (210, 212) of the tubular member (208) are axially aligned with the plurality of struts (102).

3. The implantable medical device of claim 1, wherein the geometry of the plurality of struts (102) changes in response to at least one of a length change of the frame (100) and a circumferential change of the frame (100), and the strength fibrils (210, 212) are configured to orient with the plurality of struts (102) in a direction extending toward the proximal end (104) of the frame (100) and a direction extending toward the distal end (106) of the frame (100).

4. The implantable medical device of any of claims 1-3, wherein the plurality of struts (102) include a first set of struts that extend at a first pitch toward the proximal end (104) and a second set of struts that extend at a second pitch toward the distal end (106), and the strength fibrils (210, 212) include a first set of fibrils (210) that extend at approximately the first pitch toward the proximal end (104) and a second set of fibrils (212) that extend at approximately the second pitch toward the distal end (106).

5. The implantable medical device of claim 4, wherein the first set of fibrils (210) overlap with the second set of fibrils (212) throughout the tubular member (208).

6. The implantable medical device of any of claims 15 wherein the tubular member (208) is configured to allow expansion and contraction of the frame (100) in response to at least one of a length change of the frame (100), a circumferential change of the frame (100), and angular displacement of the frame (100).

7. The implantable medical device of claim 6, wherein the tubular member (208) is configured to resist residual elastic strain acting against frame (100) deformation in response to at least one of the length change of the frame (100), the circumferential change of the frame (100), and the angular displacement of the frame (100).

## Patentansprüche

1. Implantierbare medizinische Vorrichtung, umfassend:
einen Rahmen (100) mit einer Vielzahl von Streben (102), die geflochten sind und sich spiralförmig zwischen einem proximalen Ende (104) und einem distalen Ende (106) des Rahmens erstrecken; und
eine kontinuierliche Folie, die aus expandiertem Polytetrafluorethylen (ePTFE) gebildet ist und ein röhrenförmiges Element (208) definiert, das an dem Rahmen (100) befestigt ist, und Fibrillen (210, 212) beinhaltet, die dazu konfiguriert sind, mit einer Geometrie der Vielzahl von Streben zusammenzufallen, wobei die Fibrillen (210, 212) Festigkeitsfibrillen, die mit der Vielzahl von Streben (102) ausgerichtet sind und sich parallel zu dieser erstrecken, und nicht ausgerichtete Fibrillen (318), die nicht mit der Vielzahl von Streben (102) ausgerichtet sind und die Festigkeitsfibrillen (210, 212) miteinander verbinden, beinhalten, wobei die kontinuierliche Folie eine Kraft, um eine Festigkeitsrichtung zu brechen, und eine Kraft, um eine Querrichtung zu brechen, und ein Verhältnis von Festigkeitsrichtung zu Querrichtung von 30 oder mehr aufweist, sodass die Festigkeitsfibrillen (210, 212) dazu konfiguriert sind, innerhalb des röhrenförmigen Elements (208) zu scheren, um eine Ausrichtung mit der Vielzahl von Streben (102) als Reaktion auf mindestens eine von einer Längenänderung des Rahmens (100) und einer Umfangsänderung des Rahmens (100) aufrechtzuerhalten.

2. Implantierbare medizinische Vorrichtung nach Anspruch 1, wobei die Festigkeitsfibrillen (210, 212) des röhrenförmigen Elements (208) axial mit die Vielzahl von Streben (102) ausgerichtet sind.

3. Implantierbare medizinische Vorrichtung nach Anspruch 1, wobei sich die Geometrie der Vielzahl von Streben (102) als Reaktion auf mindestens eine von einer Längenänderung des Rahmens (100) und einer Umfangsänderung des Rahmens (100) ändert und die Festigkeitsfibrillen (210, 212) dazu konfiguriert sind, sich mit der Vielzahl von Streben (102) in einer Richtung, die sich zu dem proximalen Ende (104) des Rahmens (100) hin erstreckt, und einer Richtung, die sich zu dem distalen Ende (106) des Rahmens (100) hin erstreckt, zu orientieren.

4. Implantierbare medizinische Vorrichtung nach einem der Ansprüche 1-3, wobei die Vielzahl von Streben (102) einen ersten Satz von Streben, die sich in einer ersten Steigung zu dem proximalen Ende (104) hin erstrecken, und einen zweiten Satz von Streben, die sich in einer zweiten Steigung zu dem distalen Ende (106) hin erstrecken, beinhaltet, und die Festigkeitsfibrillen (210, 212) einen ersten Satz von Fibrillen (210), die sich an in etwa der ersten Steigung zu dem proximalen Ende (104) hin erstrecken, und einen zweiten Satz von Fibrillen (212), die sich an in etwa der zweiten Steigung zu dem distalen Ende (106) hin erstrecken, beinhalten.

5. Implantierbare medizinische Vorrichtung nach Anspruch 4, wobei der erste Satz von Fibrillen (210) mit dem zweiten Satz von Fibrillen (212) im gesamten röhrenförmigen Element (208) überlappt.

6. Implantierbare medizinische Vorrichtung nach einem der Ansprüche 1-5, wobei das röhrenförmige Element (208) dazu konfiguriert ist, eine Expansion und Kontraktion des Rahmens (100) als Reaktion auf mindestens eine von einer Längenänderung des Rahmens (100), einer Umfangsänderung des Rahmens (100) und einer Winkelverschiebung des Rahmens (100) zu ermöglichen.

7. Implantierbare medizinische Vorrichtung nach Anspruch 6, wobei das röhrenförmige Element (208) dazu konfiguriert ist, einer elastischen Restbelastung, die gegen eine Verformung des Rahmens (100) wirkt, als Reaktion auf mindestens eine von der Längenänderung des Rahmens (100), der Umfangsänderung des Rahmens (100) und der Winkelverschiebung des Rahmens (100) zu widerstehen.

## Revendications

1. Dispositif médical implantable comprenant :
un cadre (100) comportant une pluralité d'entretoises (102) qui sont tressées et s'étendent de manière hélicoïdale entre une extrémité proximale (104) et une extrémité distale (106) du cadre ; et
un film continu formé de polytétrafluoroéthylène expansé (PTFEe) définissant un élément tubulaire (208) fixé au cadre (100), et comprenant des fibrilles (210, 212) qui sont conçues pour coïncider avec une géométrie de la pluralité d'entretoises, lesdites fibrilles (210, 212) comprenant des fibrilles de résistance qui sont alignées et s'étendent parallèlement à la pluralité d'entretoises (102) et des fibrilles non orientées (318) qui ne sont pas alignées avec la pluralité d'entretoises (102) et relient les fibrilles de résistance (210, 212) ensemble, le film continu présentant une force destinée à rompre la direction de résistance et une force destinée à rompre la direction transversale et un rapport de la direction de résistance à la direction transversale supérieur ou égal à 30 de sorte que les fibrilles de résistance (210, 212) soient conçues pour se cisailler à l'intérieur de l'élément tubulaire (208) de manière à maintenir l'alignement avec la pluralité d'entretoises (102) en réponse à au moins un changement parmi un changement de longueur du cadre (100) et un changement de circonférence du cadre (100).

2. Dispositif médical implantable selon la revendication 1, lesdites fibrilles de résistance (210, 212) de l'élément tubulaire (208) étant alignées axialement avec la pluralité d'entretoises (102).

3. Dispositif médical implantable selon la revendication 1, ladite géométrie de la pluralité d'entretoises (102) changeant en réponse à au moins un parmi un changement de longueur du cadre (100) et un changement de circonférence du cadre (100), et lesdites fibrilles de résistance (210, 212) étant conçues pour s'orienter avec la pluralité d'entretoises (102) dans une direction s'étendant vers l'extrémité proximale (104) du cadre (100) et une direction s'étendant vers l'extrémité distale (106) du cadre (100).

4. Dispositif médical implantable selon l'une quelconque des revendications 1 à 3, ladite pluralité d'entretoises (102) comprenant un premier ensemble d'entretoises qui s'étendent selon un premier pas en direction de l'extrémité proximale (104) et un second ensemble d'entretoises qui s'étendent selon un second pas en direction de l'extrémité distale (106), et lesdites fibrilles de résistance (210, 212) comprenant un premier ensemble de fibrilles (210) qui s'étendent suivant approximativement le premier pas en direction de l'extrémité proximale (104) et un second ensemble de fibrilles (212) qui s'étendent selon approximativement le second pas en direction de l'extrémité distale (106).

5. Dispositif médical implantable selon la revendication 4, ledit premier ensemble de fibrilles (210) chevauchant ledit second ensemble de fibrilles (212) à travers l'élément tubulaire (208).

6. Dispositif médical implantable selon l'une quelconque des revendications 1 à 5, ledit élément tubulaire (208) étant conçu pour permettre l'expansion et la contraction du cadre (100) en réponse à au moins un parmi un changement de longueur du cadre (100), un changement de circonférence du cadre (100) et un déplacement angulaire du cadre (100).

7. Dispositif médical implantable selon la revendication 6, ledit élément tubulaire (208) étant conçu pour résister à une contrainte élastique résiduelle agissant contre la déformation du cadre (100) en réponse à au moins un parmi le changement de longueur du cadre (100), le changement de circonférence du cadre (100) et le déplacement angulaire du cadre (100).
